# EUROPEAN PATENT APPLICATION

(11) **EP 2 599 492 A1**
(43) Date of publication of application: **05.06.2013**
(21) Application number: 12194238.7
(22) Date of filing: 26.11.2012
(51) Int. Cl.: A61K 36/062

(54) **DHEA production-promoting agent and method for producing DHEA production-promoting agent**

(30) Priority: 29.11.2011 JP 2011261075
(71) Applicant: Niigata Beer Corporation, Niigata 953-0012 (JP)
(72) Inventor: Usami, Ken, Niigata-shi, Niigata 953-0012 (JP); Konishi, Tetsuya, Niigata-shi, Niigata 956-8603 (JP); Nakagawa, Saori, Niigata-shi, Niigata 956-8603 (JP); Yamato, Susumu, Niigata-shi, Niigata 956-8603 (JP); Nishida, Hiroshi, Niigata-shi, Niigata 956-8603 (JP)
(74) Representative: Isarpatent

(57) **Abstract**

The present invention aims to provide novel DHEA production promoting agent for anti-aging and improvement of life functions, and applied products that contain the agent. The applied products include foods, food additives, supplements, lotions, cosmetics, etc.

The DHEA production promoting agent of the present invention contains an extract of truffles. It is desirable that this extract is at least one of extraction liquids extracted with an organic solvent or hot water. More preferably, it is obtained by extracting the truffles with the organic solvent at the temperature range of 50 C° to 80 C°. Moreover, it is desirable to use the artificially-cultivated truffles.

## Description

### [Technical Field]

The present invention relates to a promoting agent for producing dehydroepiandrosterone (referred to as "DHEA", hereinafter) and its usage. More specifically, the present invention relates to DHEA production-promoting agent containing truffle extracts and its usage.

### [Background Art]

DHEA is a sex steroid hormone secreted by the adrenal cortex. More than 99% of DHEA becomes sulfide in the blood and exists as dehydroepiandrosterone sulfide (hereinafter, referred as "DHEA-s"). Generally, these DHEA and DHEA-s are referred as adrenal androgens and hereinafter, both are also collectively referred to as "DHEA". DHEA is a sex hormone, amount of which fluctuates over time depending on the age. It is known that the amount is low before the age of puberty, reaches the peak during the puberty, and then, decreases with time.

The efficacy of DHEA is known, for example, as the promoting effect of keratinization of epidermis, and the barrier effect of skin as well as the prevention of deterioration, such as sagging skin, wrinkles and luster, and therefore, it is reported for DHEA to be effective against the aging. In addition to this, the effects such as anti-atherogenic action, anti-diabetic effect, anti-osteoporosis action and anti-obesity effect have been also reported as its efficacy.

For this reason, it is assumed that DHEA may be useful to prevent and improve various diseases and against aging, thus a direct administration of DHEA as well as an indirect way to promote the production of DHEA has been attempted. As the former "direct" administration method, for example, a method of a direct administration of DHEA, and a method of a direct administration of DHEA precursor (Diosgenin), which produces DHEA once being metabolized in the body, are disclosed in Patent Document 1. As for the latter "indirect" administration method, the promotion of production of DHEA by the transdermal absorption, through the skin, and nasal mucosa, of a composition consisting of rose oil, valerian oil, dimethoxymethyl benzene and linalool, has been disclosed in Patent Document 2. DHEA production promoting agent, made from powders of fish-scales, is also disclosed in Patent Document 3. Furthermore, DHEA production promoting agent made from extracts obtained by mixing dry ingredients, of chamomile (Chamaemelum nobile), Houttuynia cordata, grapes, and Crataegus oxyacantha, has been disclosed in Patent Document 4.

However, there is a possible risk to upset the hormone balance in directly administrating sex hormones such as DHEA or DHEA precursor. On the other hand, in the case of administration of DHEA production promoting agent, the transdermal absorption, as described above, poses a difficulty in achieving the sufficient amount of absorption. In case of the powders of fish-scales, the oral application may be considered; however, there is a problem with the taste. In case of the mixed composition of Chamaemelum nobile, Houttuynia cordata, grapes and Crataegus oxyacantha, not only there is a need of procurement of the several ingredients, but also it must be mixed in predetermined proportions, hence it remains problematic for production.

The truffles, an expensive ingredient, are known to have a variety of bioactive components, and their effects on suppressing the damage caused by the living and natural environments are known. For example, in Patent Document 5, it is disclosed that the truffle extracts, the truffle fruiting body and fungal form extracted by a solvent such as ethanol, are to be effective in suppressing the biological aging. In Patent Document 6, it is revealed that the truffle extracts are also effective in moisturizing of the skin as well as the prevention of immunological deterioration. In Patent Document 7, it is disclosed that the truffle extracts have the effect of the anti-hyperlipidemia, improvement of the immune balance as well as the secretagogue action of a secretory immunoglobulin A (IgA).

However, there is no reported case so far, to validate if the truffle itself contains DHEA or a DHEA precursor, or if the truffle has an ability to increase the concentration of DHEA (that is, the promotion of the production of DHEA) in the blood when it is consumed.

### [Prior Art Documents]

### [Patent Document]

[Patent Document 1] JP Patent Publication (unexamined) 2007-16013
[Patent Document 2] JP Patent Publication (unexamined) 2007-8862
[Patent Document 3] JP Patent Publication (unexamined) 2005-306761
[Patent Document 4] JP Patent Publication (unexamined) 2008-231031
[Patent Document 5] JP Patent Publication (unexamined) 2002-249438
[Patent Document 6] JP Patent Publication (unexamined) 2006-69969
[Patent Document 7] JP Patent Publication (unexamined) 2010-222265

### [Summary of the Invention]

### [The purpose of the Invention]

The present invention has been made in view of such circumstances, overcoming the flaws of the conventional DHEA production-promoting agent. A purpose of the invention is to provide a new type of DHEA production-promoting agent, and the applied products including the agent, for example food, food additives, supplements, lotions, cosmetics and etc., which work effectively in improving the living function as well as against the aging,

After extensive studies by the inventors, it is found that the extract of truffle has the capacity to promote the production of DHEA, and such a discovery has led to a completion of the present invention.

### [Procedures to realize the Purpose]

That is, the present invention is exemplified by aspects below having the following components and features.

### (Aspect 1)

DHEA production promoting agent, comprising an extract of a truffle.

### (Aspect 2)

A method for producing DHEA production promoting agent, comprising steps of: preparing a truffle as a ingredient for said agent; and
extracting said truffle.

### (Aspect 3)

The method for producing the DHEA production promoting agent according to aspect 2, wherein said truffle is extracted with organic solvent or hot water in said extracting step.

### (Aspect 4)

The method for producing DHEA production promoting agent according to aspect 3, wherein said organic solvent is a lower monohydric alcohol and temperature of said alcohol is set within a range of 50 C° to 80 C°.

### (Aspect 5)

The method for producing DHEA production promoting agent according to any one of aspects 2 to 4, wherein said truffle is a mycelium or a fruiting body with an amorphous form and said mycelium and said fruiting body are artificially cultured.

### (Aspect 6)

The method for producing DHEA production promoting agent according to any one of aspects 2 to 5, wherein said truffle is cultivated with a medium and said medium comprising a mixture of at least one carbohydrate source selected from the group consisting of rice bran, wheat bran, and glucose, and at least one edible ingredient selected from the group consisting of burdock, nuts, and berries.

### (Aspect 7)

The method for producing DHEA production promoting agent according to any one of aspects 2 to 6, further comprising steps of:
drying said truffle; and
grinding said truffle.

### (Aspect 8)

DHEA production promoting agent obtainable according to any one of aspects 2 to 7.

### (Aspect 9)

Food product comprising DHEA production promoting agent according to any one of aspect 1 or 8.

### (Aspect 10)

Food additive comprising DHEA production promoting agent according to any one of aspect 1 or 8.

### (Aspect 11)

Nutritional supplement comprising DHEA production promoting agent according to any one of aspect 1 or 8.

### (Aspect 12)

Lotion comprising DHEA production promoting agent according to any one of aspect 1 or 8.

### (Aspect 13)

Bath agent comprising DHEA production promoting agent according to any one of aspect 1 or 8.

### [Effect of the Invention]

According to the present invention, for example, by oral administration of the products of the present invention into humans or animals, the amount of production of DHEA in the blood can be increased. In addition, since DHEA has aforementioned benefits, the production of DHEA can be increased by consuming the DEHA production-promoting agent or its applied products of the present invention. Therefore, it can be utilized to obtain various effects on, for example, anti-aging, improvement of the skin, as well as immunostimulatory action.

In addition, the DHEA production promoting agent of the present invention utilizes an extract of the truffles as one of the ingredients, thus, it excels in terms of aroma and flavor. Moreover, there is no need in using mixture of multiple plants as ingredients. Furthermore, as will be described later, the truffle itself contains an oleic acid, which is effective for supply of vitamin D, elimination of an active oxygen (especially singlet oxygen), anti-allergic effect, as well as anti-aging. Therefore, it is expected for the added value of the present invention to be increased, by demonstrating the various effects, such as anti-aging, in a more pronounced and synergistic manner, due to the presence of the oleic acid and the increase of DHEA production,.

In addition, instead of using the natural truffle fruiting body regarded as high market value ingredient, by using, as ingredients, a truffle fruiting body with atypical (amorphous) form, valued low in the market, and an artificially cultivated truffle fruiting body and mycelium, it allows to improve the productivity in production of the agent of the present invention as well as to contribute to the cost reduction.

In addition to the enhancing the aforementioned effect of production of DHEA, it is also expected to ensure an additional benefit on the antioxidant effect to the humans or animals who consume the DHEA production promoting agent, containing an extract of truffles, cultivated by the medium mixed with the carbohydrate source such as rice bran and the edible ingredient such as burdock, instead of the usual medium consisting of wood sawdust.

### [Brief Description of the Drawings]

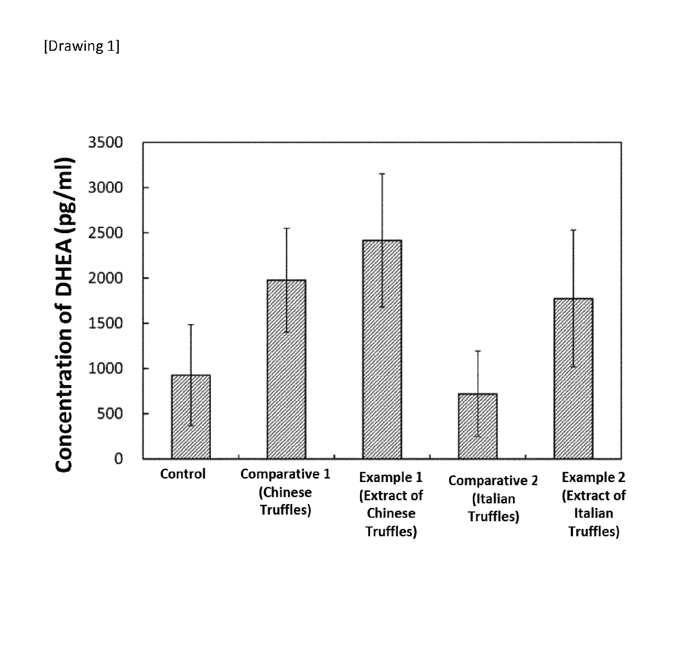

Figure 1 is a graph demonstrating the concentration of DHEA in blood plasmas of rats, to whom truffles or truffle extracts are administered.

### [Detailed Description of the Embodiments for Implemening the Invention]

Hereinafter, the embodiments of the present invention will be described in detail.

(As for the truffle, the ingredient of the present invention)
Truffle, used as an ingredient in the present invention, is preferably a fungi belonging to the Ascomycota Tuberaceae. There is no particular restriction, however it is preferred to use the white truffles (Tuber magnatum) and/or black truffles (Tuber melanosporum and Tuber indicum). It is also possible to use Tuber aestivum, Tuber borchi, Tuber macrosporum, Tuber masentericum and Tuber unciatum. The truffle grows in clumps under the ground, and its hymenium is not open to the outside. Its fruiting body possesses very strong smell and, in many cases, animals such as squirrels and rabbits dig up the fruiting body from the ground to eat it.

As for the truffles utilized in the present invention, in addition to the natural truffles, for example, it is also possible to use the artificially-cultivated fruiting bodies, liquid-cultured mycelium as well as their processed products. Since the production conditions and process can be more stable, easier to manage and less expensive, the artificially-cultivated truffles are more desirable for the purpose of this present invention, comparing to the natural truffles.

The strain (fungus) can be collected by an aseptic operation in a suitable manner from truffles. For example, it is possible to obtain the fungus by aseptically cutting out a portion of an internal tissue from a raw fruiting body.

The cultivation of fruiting bodies is performed as follows. A culture medium, such as a medium for the edible fungus, can be used in this case. It is publicly known that the medium, made by mixing the sawdust (a piece of wood or wood chips), the rice bran and other nutrients, is used for the cultivation of the fruiting body. The strain is injected into a hole drilled in the culture medium, and it is cultured continuously at a low temperature. Then, fungal hyphae of white fluff on the entire medium are grown in about a week. The hyphae are spread around in one month or two months. After three or four months, fruiting body primordia of about 2 to 3 cm are formed on the surface of the medium covered with white aerial mycelium.

The sawdust, a commonly used medium for the truffle cultivation, can be used in the present invention, however, it is also possible to use the mixture described below. That is, the mixture with an edible ingredient containing at least one of burdock, nuts and berries, and an carbohydrate source containing at least one of rice bran, wheat bran or glucose. The present inventors have known that, based on their experiences, the truffles cultivated by the medium containing the aforesaid mixture combined with the truffles strain have a higher value in the antioxidant capacity (ORAC: Oxygen Radical Absorbance Capacity) than the truffles normally cultivated by the medium containing the sawdust. In terms of the weight ratio of the mixture for the antioxidant effect, useful to the living body, it is preferable to mix the carbohydrate source and the edible ingredient with the weight ratio of the edible ingredient to the carbohydrate source to be 0.5 to 5. As for the edible ingredient, it is preferable to use a burdock grounded to a granular. Consequently, by consuming the DHEA production promoting agent of the present invention using the extract of the truffles cultivated by the medium described in the latter method, it is expected for humans and animals to obtain an additional benefit of antioxidant capacity to the enhanced production of DHEA.

As for the mycelium, for example, it can be obtained by liquid culture as described below.
In general, it is possible to use the medium of filamentous fungi (heterotrophic bacteria saprophytic type) for the liquid cultivation. Preferably, for the source of nitrogen, a yeast extract, a malt extract, a corn steep liquor, a dried yeast and its chaff, peptone, bouillon, various amino acids, various ammonium salts and ammonium compounds can be used. As for the source of carbons, starch obtained from natural extracts products, dextrin, oligosaccharides, as well as glucose used generally for medium, xylose, sucrose, maltose, galactose and mannose are desirable. Salts added to the medium can be preferably magnesium sulfate, dipotassium hydrogen phosphate, ammonium dihydrogen phosphate or ammonium sulfate. In liquid culture, the mycelia are grown with the passage of culture days, however, after the amount of mycelium reached the maximum value, a part of the mycelium yield starts to dissolve itself by over-cultivation and the amount will be reduced. As a result, it is not advisable to continue the cultivation unnecessarily and it is better to recover the mycelia at the time of maximum yield.

As described above, not only the mycelium or truffle fruiting body, it is also possible to use the enzyme-treated mycelium and truffle fruiting body and its residue as the medium. As for the enzyme, it is possible to use enzymes with a substrate of polysaccharide that makes up the cell wall, and other enzymes with a substrate of the other constituent. For example, glucanase, chitinase, protease, pectinase, lipase, cellulase, xylanase, mannanase, hemicellulase, nuclease and the like can be used and possibly mixed for use. Additionally, it is not particularly specified as the origin of the enzyme, for example, Aspergillus niger, Aspergillus melleus, Aspergillus oryzae, Rhizopus niveus, Bacillus subtilis, Arthrobacter sp., Trichoderma viride are recognized as the preferred origin.

(As for truffle extract of the present invention)
The fruiting body, the liquid cultured mycelium and its enzyme treated materials become the truffle extract of the present invention by going through the following operation such as drying, grinding and extracting.

As the drying method, there is no restriction, however, it can be mentioned the following methods, such as natural drying, heat drying, blast drying, steam drying, spray drying, freeze-drying and the likes. The freeze-drying method causes no deterioration due to heat and allows easy pulverization and an excellent palatability to be obtained. Therefore, it is desirable to be used. If necessary, it is easier to grain the truffles if the truffle fruiting body and mycelia are cut into about 2cm size or less, after they are collected and washed. In the case of the freeze-drying, it should be done in the environment under -20C°, for example, the freezing process should take place in a known freezer set to -20 C° or less. In this freezing process, as not to cause ice crystals, the freezing process should be set to -30 C° or less, and more preferably, it is ideal to rapidly freeze it in the environment set to -40C° or less. After the freezing process, the freeze-drying of the truffles is completed with vacuum drying.

In the next operation of the grinding, the obtained truffles by the freeze-drying would be grinded to be fine. As for the method of the grinding is not particularly limited, as long as it is a known method, for example, the truffles may be finely cut by a cutter or finely milled by a mill, roller or grinder. As for the method of grinding, it can be determined by the purpose and the kind of blending with the truffles, there is no particular limitation on the particle size.

Secondly, as for the extracting method of the present invention, while it is not limited, but a continuous manner or a batch manner may be used, to make it possible in performing cold or warm immersion for any given duration. It is also possible to extract them more efficiently by subcritical conditions. For example, the extraction may be done by soaking the above fruiting body, the solvent-cultivated mycelia or their dried forms, in the extracting solvent for more than few minutes, preferably more than 1 hour, at a room temperature. Or the extraction may be done by heating or heat-refluxing them with the extracting solvents for more than a few minutes, preferably more than 1 hour. Then extract may be finally obtained by removing the extraction residue by filtration.

As for the extracting solvents, for example, there are aqueous solvents such as water and organic solvents as mentioned below. Here, the organic solvents include lower monohydric alcohols (methanol, ethanol, 1-propanol, 2-propanol, 1-butanol, 2-butanol, etc.), liquid polyhydric alcohols (1, 3-butylene glycol, propylene glycol, glycerin, etc.), ketones (acetone, methyl ethyl ketone, etc.), acetonitrile, esters (ethyl acetate, butyl acetate, etc.), hydrocarbons (hexane, heptane, liquid paraffin, etc.), ethers(ethyl ether, tetrahydrofuran, propyl ether etc.).

Of these, from the viewpoint of the effective production of DHEA, a preferable extracting solvent is a monohydric alcohol or a hot water. Particularly, more preferable is a lower monohydric alcohol, which is set to a temperature range from 50 C° to 80 C°. These solvents may be used by mixing two or more kinds in combination.

The adding ratio of the truffle ingredient and the extracting solvent is so that 1 g of the ingredients is to be added while 0.01 - 100 L (from 0.01 L to 100 L) of the extraction solvent to be added. When the confirmation of quality of the substance after the addition is desired to be performed by a mass analysis such as GC-MS (later described), the above adding ratio should be that the ingredients are to be 1 g while the extracting solvent to be 0.01 - 1 L, or more preferably, 20 ml, considering the burden of GS-MS.

The immersion period of the ingredients in the extracting solvent varies depending on the kinds or amounts of ingredients or the extracting solvent. However, it is desirable to be 10 minutes or more for soaking 1 g of ingredients to 1 L of the extract solvent, and more desirably, it should be 30 minutes or more and 12 hours and less. Also, under the room temperature, the immersion period should be about 10 hours to 12 hours.

The extract as obtained, that is, the one with the extracted solution, can be used as a DHEA production-promoting agent. Or, if necessary, it can be also used an extract added a process such as concentration, dilution, filtration, and decolorization or deodorization with an activated carbon. Furthermore, it can be used the dried substance derived from the extracted solution processed by concentrated drying, spray drying, or freeze-drying, to produce a DHEA production promoting agent.

The form of an extracted material of the present invention is not limited and may be, for example, a solution, paste or powder. It can be chosen, in an appropriate manner depending on the application and form of DHEA production promoting agent, as it is later described.

The DHEA production promoting agent in this presented invention requires to contain the truffle extracts, regardless of its form, it could be solid, liquid, gel or other forms. Some examples of the form are ampoules, capsules, pills, tablets, powders, granules and the like. Also, in addition to the above extracted materials, the DHEA production promoting agent in this present invention may include various additives such as excipients.

The above described DHEA production promoting agent can be used directly into a food, a food additive, a supplement, a lotion or a bath agent. Furthermore, as long as the effect of the DHEA production promoting agent is not impaired, the agent may be also used with a lipid, a wax, a hydrocarbon, a fatty acid, an alcohol, an ester, a surfactant, a metal soap, an adjusting agent pH, a preservative, a perfume, a humectant, powders, an ultraviolet absorber, a thickener, a pigment, an antioxidant, a chelating agent, an excipient, a stabilizer, a preservative, a binder, and a disintegrating agent, which are substances used in foods, cosmetics, quasi-drugs, and pharmaceuticals.

The food, the food additive, the supplement, the lotion and the bath agent of the present invention may also be in various forms besides the liquid solution state, or the dry powders as mentioned above.

The DHEA production promoting agent of the present invention may be used as an additive to foods, for example, sweets and candies, chocolates, gums, baked goods, rice crackers, and ice creams, or another example is seasoning agents such as toppings, spices, dressings, soy sauce, ketchup, mayonnaise and sauces, or dairy products such as cheese, butter, whipped cream, and margarine, or grain products, such as bread, rice, and noodles, beverages such as juices and sports drinks, and energy drinks, beers, malt beers, liqueurs, sake, shochu, whiskeys and other alcoholic beverages. The product may be consumed as it is, depending on its shape or consumer's preference , for example, by topping it on the rice or noodles. Or it may be consumed after mixed with water, hot water or milk. Also it can be blended into processed foods such as sausages, processed egg products, processed meat products, stew, curry, salad and soup.

Also, as for the supplement of the present invention, it can be used as, but not limited to, tablets, capsules, powders, granules, pills, solutions, emulsions, suspensions, drug solutions, agents of alcohol (spirits), syrups, extracts, and elixirs. Persistent or sustained-release supplements may also be produced as the supplements of the present invention. These may be, depending on the preferences or applications, served as liquid product. Or it may be provided in the form of capsules such as hard capsules and soft capsules, tablets, pills, sugar-coated tablets, powders, granules, tea-like, tea bags, or the like.

The lotion of the present invention may be used, depending on its application, for example, in a form of emulsions, creams, or facial masks.

The bath agent of the present invention may be provided in a form, not limited to, a powder, a solid, or a liquid.

Since the DHEA production promoting agent of the present invention has no special odor, (that is, it is odorless or leaves the aromatic truffles scents), when it is blended with the food, it does not expunge the flavor of the food and does make the food have excellent nutrition.

As mentioned above, the DHEA production promoting agent of the present invention can be used widely in technical fields of foods in general and health or nutrition food products, as well as in a medical field. In the usage as an article of food, as described above, it can be used in exactly the same way in normal food additives. For example, when used as an article of food, it can be heated or dissolved, or added or mixed with other food items, or it may be simply eaten as it is.

According to the DHEA production-promoting agent of the present invention or the applied products including this agent, it is possible to increase the production amount of DHEA in the blood of an animal or a person who has ingested it by oral administration or any other ways of administration. In addition, since DHEA has various effects as described above, the intake of the DHEA production-promoting agent of the present invention will increase the DHEA production. For example, it will provide the effective results on improving skins, the immunostimulatory activity, and the anti-aging.

In addition, the DHEA production-promoting agent of the present invention utilizes truffle extracts as its ingredient. Therefore, it is excellent in terms of aroma and flavor. Furthermore, it is not necessary to use a mixture of various plants as its ingredient. As will be described later, since the truffles itself are rich in oleic acid, which is effective for prevention and improvement of the diseases associated with the adult lifestyles habits, such as heart disease, high blood pressure, hardening of the arteries, as well as vitamin D, which is effective in anti-obesity action and anti-osteoporosis action, it is expected to provide more pronounced and synergetic effects on the anti-aging, etc.

The following examples are presented to demonstrate the effects of the present invention; and these have no intention of limiting the present invention thereof.

(Profiling and Identification of the Truffle Constituents)
Firstly, in order to examine whether or not the truffle extract has the DHEA constituents, fruiting bodies of the black truffles from China and Italy as well as mycelium of truffles from Italy, were prepared and extracted with ethanol (the extract criteria). Each ethanol extract was analyzed by gas chromatography mass spectrometry method (GC/MS). In performing the analysis, we separated the extract into 15 major components, and identified each of the components with the National Institute of Standards and Technology (NIST) Library, where 200000 databases were accumulated.

As a result, the truffle extracts have steroids such as brassicasterol and ergosterol, and fatty acid such as oleic acid and arachidic acid (eicosanoic acid), as their major components. Thus, the presence of such components indicates that the truffle itself is promising as a substance for supplying of vitamin D, for scavenging active oxygen (especially singlet oxygen), for providing anti-allergic as well as anti-aging and it shall be synergistic and complementary fit with DHEA production enhancing effect, which will be described later.

However, DHEA, which is a kind of steroids, was not detected in the identification and verification. Even with an additional ELISA measurement, which has a higher sensitivity than the GC/MS, it was not detected. Therefore, considering the facts that the truffle extracts did not contain significant amount of DHEA, and that the concentration of DHEA in rats ingesting the truffle extracts was increased as the results described later, we understand that, it is not that the DHEA concentration in the blood plasma being increased due to the DHEA contained in the truffle extract ingested by humans or animals, but it is inferred that some other components in the truffle extracts ingested in the body do enhance the production of DHEA, which the human or animal produces.

(Evaluation of the Concentration of DHEA in Rats Blood Plasma)
We have conducted animal experiments to validate the effect of DHEA production. Specifically, we evaluated and compared the concentrations of DHEA in the blood plasmas of rats by changing the conditions of their consumption.

(Comparative Examples)
We prepared Chinese black truffles and Italian black truffles. A predetermined amount of the powder form of these truffles (50 mg / kg-body weight) was orally administered to the rats. The blood plasmas taken from these rats are each referred to Comparative Example 1 and Comparative Example 2. In addition, as a control subject of the validation, we also prepared and evaluated the blood plasma of a mouse, which was given only distilled water, instead of the truffles.

(Practical Examples)
In addition, to create the truffle extracts, we soaked 0.1 g of dry powders of Chinese or Italian black truffle, described above, for 1 hour in 100 cc of alcohol heated and kept to a temperature of approximately 70 C°, and the same amount as the comparative examples described above, (50 mg / kg-body weight) was administered orally to rats. Blood plasmas taken from these rats are referred as Example 1 and Example 2.

(Adjustment of the Blood Plasma for the Measurement of Concentrations of DHEA)
The blood was collected 2 hours after the oral administration to the rats in each condition described above, and an anti-caking heparin was added, and then a centrifugal separation using a swing rotor at 3000rpm for 15 minutes, under its environmental temperature of 4 C°, was performed to each of the blood. 120µl of the supernatant collected from the blood in such a method was mixed well with the same amount of ethyl acetate. Since the DHEA component in the supernatant is transferred to the ethyl acetate, we succeeded in obtaining just the DHEA component by collecting the ethyl acetate and evaporating it by spraying nitrogen.

(Method for Measuring the Concentration of DHEA)
We evaluated the DHEA concentration of the above comparative samples using the ELISA method (enzyme-linked immunosorbent assay), which is one of the EIA methods (enzyme immunoassay), for quantifying the substance using the chromogenic reaction of the enzyme in the antigen-antibody reaction. More specifically, we used a commercially available DHEA EIA Kit, (manufactured by Enzo Life Science, Inc.,).

Figure 1 shows the evaluation results of the concentration of DHEA. The measurement results of each administration condition in the figure show the average value of three measurements, respectively.

Based on the evaluation results, it was found that the concentration of DHEA in the blood plasma of the rats, given the oral administration of the truffle extracts of the present invention, tended to increase. Although there are some differences of degree, such a trend of increase was confirmed in both practical examples (example 1 and example 2) of the extracts made of Chinese and Italian truffles. In other words, the fungi belonging to the family (truffle) of Ascomycota Tuberaceae, regardless of the origin of the country, it is expected to have a certain level of DHEA production promoting effects.

(Production of Food (a low-malt beer) of the present invention)
Next, we produced a low-malt beer as an example for the food including the aforementioned DHEA production promoting agent. After the completion of the following three processes, a preparation process by adding hops into 24 % by weight of malt, 30 % by weight of barley, 46% by weight of liquid sugar, and a saccharification process to simmer this prepared-liquid for 1 hour at 60 C°, and a fermentation process to ferment it for one week after adding yeast, and before a final process of heat sterilization and bottling, a predetermined amount (that is, 5 ml per 500 cc of sparkling liquor) of the truffle extraction liquid (that is, DHEA production promoting agent) of the present invention was injected. As for the truffle extraction liquid, 50 g of the mycelia was prepared. It was produced by extracting the mycelia in an alcohol with concentration of 75 % heated at about 70 C° temperature for about 1 hour, and filtering the liquids afterwards.

### [Industrial Applicability]

Owing to the present invention, it is now possible to offer the truffles, known as a delicacy, as a novel functional food to the biological body for anti-aging and the life improving function. As the ingredient of the DHEA production promoting agent of the present invention, an artificially-cultivated truffle with excellent productivity and low cost may be used. In addition, it is also possible not only to use a truffle fruiting body, which is considered as a luxury food, but also an amorphous form of fruiting body and a mycelium in culture of a fruiting body may be used as the ingredient.

Applications for the DHEA production-promoting agent of the present invention, as described above, may include functional foods, food additives, functional alcoholic beverages (e.g., low-malt beers, liqueurs), functional cosmetics, and pharmaceuticals, and therefore the agent may be also applied in many fields. Consequently, the present invention has a very high value for the industrial application.

## Claims

**1.** An extract of a truffle for use as an agent for promoting dehydroepiandrosterone (DHEA) production.

**2.** A method for producing DHEA production promoting agent, comprising steps of:
preparing a truffle as a ingredient for said agent; and
extracting said truffle.

**3.** The method for producing the DHEA production promoting agent according to claim 2, wherein said truffle is extracted with organic solvent or hot water in said extracting step.

**4.** The method for producing DHEA production promoting agent according to claim 3, wherein said organic solvent is a lower monohydric alcohol and temperature of said alcohol is set within a range of 50 C° to 80 C°.

**5.** The method for producing DHEA production promoting agent according to any one of claims 2 to 4, wherein said truffle is a mycelium or a fruiting body with an amorphous form and said mycelium and said fruiting body are artificially cultured.

**6.** The method for producing DHEA production promoting agent according to any one of claims 2 to 5, wherein said truffle is cultivated with a medium and said medium comprising a mixture of at least one carbohydrate source selected from the group consisting of rice bran, wheat bran, and glucose, and at least one edible ingredient selected from the group consisting of burdock, nuts, and berries.

**7.** The method for producing DHEA production promoting agent according to any one of claims 2 to 6, further comprising steps of:
drying said truffle; and
grinding said truffle.

**8.** DHEA production promoting agent obtainable according to any one of claims 2 to 7.

**9.** Use of an extract of a truffle as an agent for promoting production of DHEA according to claim 1 or 8 in a food product.

**10.** Use of an extract of a truffle as an agent for promoting production of DHEA according to claim 1 or 8 in a food additive.

**11.** Use of an extract of a truffle as an agent for promoting production of DHEA according to claim 1 or 8 in a nutritional supplement.

**12.** Use of an extract of a truffle as an agent for promoting production of DHEA according to claim 1 or 8 in a lotion.

**13.** Use of an extract of a truffle as an agent for promoting production of DHEA according to claim 1 or 8 in a bath agent.
